# EUROPEAN PATENT APPLICATION

(11) **EP 4 686 493 A1**
(43) Date of publication of application: **04.02.2026**
(21) Application number: 25192665.5
(22) Date of filing: 29.07.2025
(51) Int. Cl.: A61N 1/05, A61N 1/372, A61N 1/375

(54) **BIOSTIMULATOR TRANSPORT SYSTEM HAVING PROTECTIVE SLEEVE**

(30) Priority: 01.08.2024 US 202463678462 P; 28.07.2025 US 202519283025
(71) Applicant: Pacesetter, Inc., Sylmar, CA 91342 (US)
(72) Inventor: ARNAR, Bernhard, Sylmar, CA 91342 (US); JACKSON, Aaron, Sylmar, CA 91342 (US); BLUE, Jeremiah, Sylmar, CA 91342 (US); SACHA, Mike, Sylmar, CA 91342 (US); WEBER, Adam, Sylmar, CA 91342 (US)
(74) Representative: Barker Brettell Sweden AB

(57) **Abstract**

A biostimulator transport system (302) includes a catheter shaft (304) extending to a distal shaft end (306). The biostimulator transport system (302) includes a biostimulator coupling (308) mounted on the distal shaft end (306) to receive a biostimulator (100) having a fixation element (106). The biostimulator transport system (302) includes a protective sleeve (314) movable relative to the biostimulator coupling (308) between a protective state and an unprotective state. The protective sleeve (314) covers the fixation element (106) in the protective state. The protective sleeve (314) does not cover the fixation element (106) in the unprotective state. The protective sleeve (314) has a distal section (3104) including one or more folds that open when the protective sleeve (314) moves from the protective state to the unprotective state.

## Description

### TECHNICAL FIELD

The present disclosure relates to biostimulator transport systems. More specifically, the present disclosure relates to transport systems for delivering leadless biostimulators to a target tissue.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Conduction system pacing at locations other than the His-bundle provides an alternative to His-bundle pacing. Cardiac system pacing involves pacing past the His-bundle toward the right ventricle apex. More particularly, cardiac system pacing targets nerve tissue, such as a left bundle branch, at a location on the right ventricular septum below the His site. A leadless cardiac pacemaker can be delivered to the location by catheter-based delivery system.

### SUMMARY

Existing biostimulator transport systems have tubular sheaths to cover the leadless cardiac pacemakers that they deliver. The tubular sheaths are as long or longer than the leadless cardiac pacemakers that they cover. The tubular sheaths can interfere with system performance. For example, the tubular sheaths can be stiff and difficult to navigate to a septal wall. Also, the tubular sheaths can cause steering or actuation difficulties, such as a reduction in deflection angle that requires oversteering to correct, binding, or an inability to translate the leadless cardiac pacemaker. Furthermore, the tubular sheaths can isolate the leadless cardiac pacemakers from a conductive fluid and, thus, interfere with communication through the conductive fluid. Accordingly, there is a need for a biostimulator transport system having a protective sleeve that can transition between a protective state and an unprotective state, exposing a biostimulator, without impeding system steering or communication.

A biostimulator transport system is described. In an embodiment, the biostimulator transport system includes a catheter shaft extending to a distal shaft end. The biostimulator transport system includes a biostimulator coupling mounted on the distal shaft end to receive a biostimulator having a fixation element. The biostimulator transport system includes a protective sleeve movable relative to the biostimulator coupling between a protective state and an unprotective state. The protective sleeve covers the fixation element in the protective state. The protective sleeve does not cover the fixation element in the unprotective state. The protective sleeve includes one or more slits to expand when the protective sleeve moves from the protective state to the unprotective state.

A biostimulator transport system is described. In an embodiment, the biostimulator transport system includes a catheter shaft extending to a distal shaft end. The biostimulator transport system includes a biostimulator coupling mounted on the distal shaft end to receive a biostimulator having a fixation element. The biostimulator transport system includes a protective sleeve movable relative to the biostimulator coupling between a protective state and an unprotective state. The protective sleeve covers the fixation element in the protective state. The protective sleeve does not cover the fixation element in the unprotective state. The protective sleeve includes a cap mounted on a wire.

A biostimulator transport system is described. In an embodiment, the biostimulator transport system includes a catheter shaft extending to a distal shaft end. The biostimulator transport system includes a biostimulator coupling mounted on the distal shaft end to receive a biostimulator having a fixation element. The biostimulator transport system includes a protective sleeve movable relative to the biostimulator coupling between a protective state and an unprotective state. The protective sleeve covers the fixation element in the protective state. The protective sleeve does not cover the fixation element in the unprotective state. The protective sleeve has a corrugated wall to contract when the protective sleeve moves from the protective state to the unprotective state.

A biostimulator transport system is described. In an embodiment, the biostimulator transport system includes a catheter shaft extending to a distal shaft end. The biostimulator transport system includes a biostimulator coupling mounted on the distal shaft end to receive a biostimulator having a fixation element. The biostimulator transport system includes a protective sleeve movable relative to the biostimulator coupling between a protective state and an unprotective state. The protective sleeve covers the fixation element in the protective state. The protective sleeve does not cover the fixation element in the unprotective state. The protective sleeve is invertible to fold on itself when the protective sleeve moves from the protective state to the unprotective state.

A biostimulator transport system is described. In an embodiment, the biostimulator transport system includes a catheter shaft extending to a distal shaft end. The biostimulator transport system includes a biostimulator coupling mounted on the distal shaft end to receive a biostimulator having a fixation element. The biostimulator transport system includes a protective sleeve movable relative to the biostimulator coupling between a protective state and an unprotective state. The protective sleeve covers the fixation element in the protective state. The protective sleeve does not cover the fixation element in the unprotective state. The protective sleeve includes a sleeve body containing a compliant plug through which the biostimulator passes when the protective sleeve moves from the protective state to the unprotective state.

A biostimulator transport system is described. In an embodiment, the biostimulator transport system includes a catheter shaft extending to a distal shaft end. The biostimulator transport system includes a biostimulator coupling mounted on the distal shaft end to receive a biostimulator having a fixation element. The biostimulator transport system includes a protective sleeve movable relative to the biostimulator coupling between a protective state and an unprotective state. The protective sleeve covers the fixation element in the protective state. The protective sleeve does not cover the fixation element in the unprotective state. The protective sleeve includes a thread engaging the biostimulator coupling such that rotation of the biostimulator coupling moves the protective sleeve from the protective state to the unprotective state.

A biostimulator transport system is described. In an embodiment, the biostimulator transport system includes a catheter shaft extending to a distal shaft end. The biostimulator transport system includes a biostimulator coupling mounted on the distal shaft end to receive a biostimulator having a fixation element. The biostimulator transport system includes a protective sleeve movable relative to the biostimulator coupling between a protective state and an unprotective state. The protective sleeve covers the fixation element in the protective state. The protective sleeve does not cover the fixation element in the unprotective state. An outer sleeve surface of the protective sleeve has one or more bosses or recesses.

A biostimulator transport system is described. In an embodiment, the biostimulator transport system includes a catheter shaft extending to a distal shaft end. The biostimulator transport system includes a biostimulator coupling mounted on the distal shaft end to receive a biostimulator having a fixation element. The biostimulator transport system includes a protective sleeve movable relative to the biostimulator coupling between a protective state and an unprotective state. The protective sleeve covers the fixation element in the protective state. The protective sleeve does not cover the fixation element in the unprotective state. The protective sleeve includes one or more telescoping sections to contract when the protective sleeve moves from the protective state to the unprotective state.

A biostimulator transport system is described. In an embodiment, the biostimulator transport system includes a catheter shaft extending to a distal shaft end. The biostimulator transport system includes a biostimulator coupling mounted on the distal shaft end to receive a biostimulator having a fixation element. The biostimulator transport system includes a protective sleeve movable relative to the biostimulator coupling between a protective state and an unprotective state. The protective sleeve covers the fixation element in the protective state. The protective sleeve does not cover the fixation element in the unprotective state. The protective sleeve has a distal section including one or more folds that open when the protective sleeve moves from the protective state to the unprotective state. A biostimulator system including a biostimulator mounted on the biostimulator transport system, and methods of using the biostimulator transport system, is also described.

The above summary does not include an exhaustive list of all aspects of the present invention. It is contemplated that the invention includes all systems and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a side view of a biostimulator, in accordance with an embodiment.
FIG. 3 is a perspective view of a biostimulator system, in accordance with an embodiment.
FIG. 4 is a perspective view of a protective sleeve of a biostimulator transport system, in accordance with an embodiment.
FIG. 5 is a perspective view of a protective sleeve of a biostimulator transport system, in accordance with an embodiment.
FIG. 6 is a side view of a protective sleeve of a biostimulator transport system in a protective state, in accordance with an embodiment.
FIG. 7 is a side view of a protective sleeve of a biostimulator transport system in an unprotective state, in accordance with an embodiment.
FIG. 8 is a perspective view of a protective sleeve of a biostimulator transport system in a protective state, in accordance with an embodiment.
FIG. 9 is a perspective view of a protective sleeve of a biostimulator transport system in an unprotective state, in accordance with an embodiment.
FIG. 10 is a perspective view of a protective sleeve of a biostimulator transport system in a protective state, in accordance with an embodiment.
FIG. 11 is a side view of a protective sleeve of a biostimulator transport system in an unprotective state, in accordance with an embodiment.
FIG. 12 is a side view of a protective sleeve of a biostimulator transport system in an unprotective state, in accordance with an embodiment.
FIG. 13 is a perspective view of a protective sleeve of a biostimulator transport system in a protective state, in accordance with an embodiment.
FIG. 14 is a side view of a protective sleeve of a biostimulator transport system in an unprotective state, in accordance with an embodiment.
FIG. 15 is a side view of a protective sleeve of a biostimulator transport system in a protective state, in accordance with an embodiment.
FIG. 16 is a side view of a protective sleeve of a biostimulator transport system in an unprotective state, in accordance with an embodiment.
FIG. 17 is a side view of a protective sleeve of a biostimulator transport system in a protective state, in accordance with an embodiment.
FIG. 18 is a side view of a protective sleeve of a biostimulator transport system in an unprotective state, in accordance with an embodiment.
FIG. 19 is a perspective view of a protective sleeve of a biostimulator transport system in a protective state, in accordance with an embodiment.
FIG. 20 is a side view of a protective sleeve of a biostimulator transport system in an unprotective state, in accordance with an embodiment.
FIG. 21 is a side view of a protective sleeve of a biostimulator transport system in a protective state, in accordance with an embodiment.
FIG. 22 is a perspective view of a protective sleeve of a biostimulator transport system in a protective state, in accordance with an embodiment.
FIG. 23 is a side view of a protective sleeve of a biostimulator transport system in an unprotective state, in accordance with an embodiment.
FIG. 24 is a side view of a protective sleeve of a biostimulator transport system in an unprotective state, in accordance with an embodiment.
FIG. 25 is a side view of a protective sleeve of a biostimulator transport system in an unprotective state, in accordance with an embodiment.
FIG. 26 is a side view of a protective sleeve of a biostimulator transport system in a protective state, in accordance with an embodiment.
FIG. 27 is a perspective view of a protective sleeve of a biostimulator transport system, in accordance with an embodiment.
FIG. 28 is a perspective view of a protective sleeve of a biostimulator transport system, in accordance with an embodiment.
FIG. 29 is a side view of a protective sleeve of a biostimulator transport system in a protective state, in accordance with an embodiment.
FIG. 30 is a side view of a protective sleeve of a biostimulator transport system in an unprotective state, in accordance with an embodiment.
FIG. 31 is a perspective view of a protective sleeve of a biostimulator transport system, in accordance with an embodiment.
FIG. 32 is a perspective view of a protective sleeve of a biostimulator transport system, in accordance with an embodiment.

### DETAILED DESCRIPTION

Embodiments describe a biostimulator transport system, such as a transport system for delivering a biostimulator, having a protective sleeve. As described below, the biostimulator transport system can be used to deliver a biostimulator to a heart. The biostimulator transport system may, however, be used to deliver the biostimulator to other anatomies. Thus, reference to the biostimulator as being a cardiac pacemaker is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator transport system. Similarly, "proximal" may indicate a second direction opposite to the first direction. Such terms are provided to establish relative frames of reference, however, and are not intended to limit the use or orientation of the biostimulator transport system to a specific configuration described in the various embodiments below.

In an aspect, biostimulator transport systems include protective sleeves that transition between a protective state and an unprotective state. More particularly, a biostimulator transport system includes a biostimulator coupling to receive a biostimulator, and a protective sleeve is movable relative to the biostimulator coupling between the protective state, in which a fixation element of the biostimulator is covered for delivery to a target tissue, to the unprotective state, in which the fixation element is exposed for implantation into the target tissue. The protective sleeve can transition between the protective state and the unprotective state without interfering with steering of a catheter shaft of the system. Various embodiments of the protective sleeve are described, including a protective sleeve having one or more slits that expand when the protective sleeve transitions to the unprotective state, allowing communication signals to transmit effectively through the slit(s).

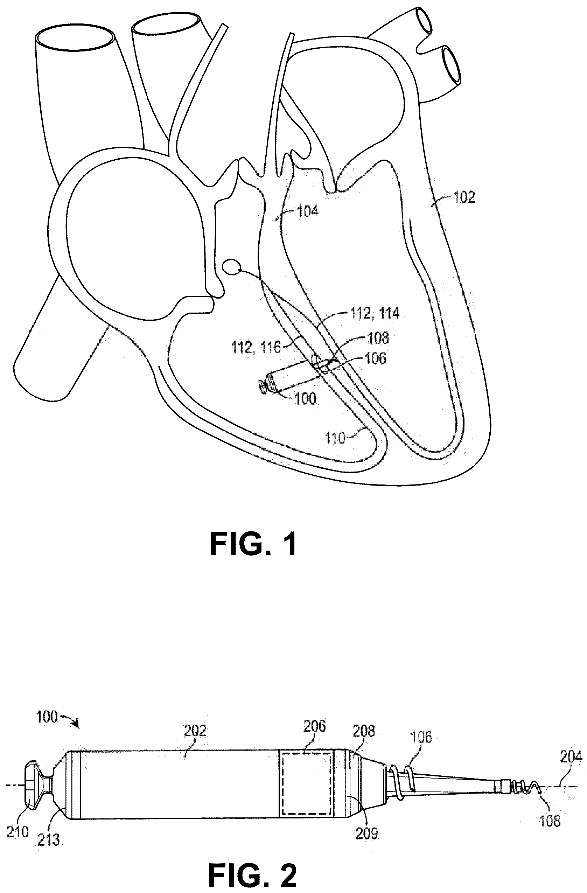

Referring to FIG. 1, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 100. The biostimulators 100 can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to a septum 104 of the heart 102. More particularly, the biostimulator 100 can be delivered to the septum 104, and one or more elements, such as a fixation element 106 and/or a pacing element 108 can pierce a septal wall 110 of the septum 104 to engage and anchor the biostimulator 100 to the tissue. In a particular embodiment, the biostimulator 100 can use two or more electrodes located on or within a housing of the biostimulator 100 for pacing the cardiac chamber upon receiving a triggering signal from at least one other device within the body. In an embodiment, one or more of the fixation element 106 or the pacing element 108 is an active electrode.

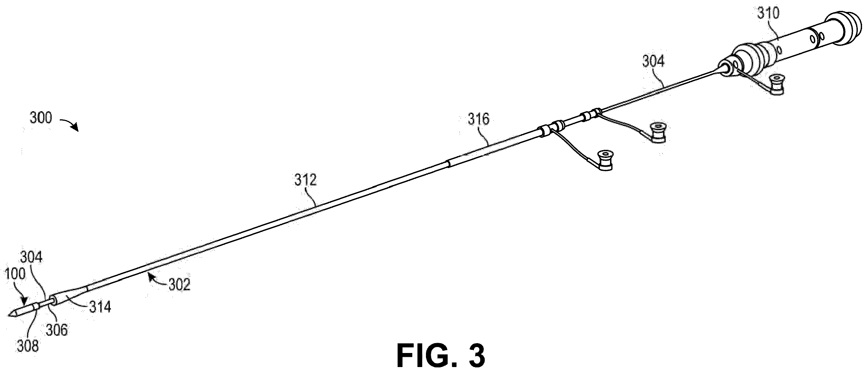

Leadless pacemakers or other leadless biostimulators 100 can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems, and are described further below. For example, in some implementations of transport systems (FIG. 3), a leadless pacemaker is attached or connected to a distal end of a catheter and advanced intravenously into or out of the heart 102. The transport system can include features to engage the leadless pacemaker to allow fixation of the leadless pacemaker to tissue. For example, in implementations where the leadless pacemaker includes an active engaging mechanism, such as a fixation element 106, the transport system can include a biostimulator coupling, e.g., a docking cap or key, at a distal end of the catheter, and the docking cap or key may be configured to engage the leadless pacemaker and apply torque to screw the active engaging mechanism into or out of the tissue. In other implementations, the biostimulator coupling of the transport system includes clips designed to match the shape of a feature on the leadless pacemaker and apply torque to screw the active engaging mechanism into or out of the tissue.

When the biostimulator 100 is delivered to and screwed into the septum 104 of the heart 102, the pacing element 108 and/or the fixation element 106 may be positioned for deep septal pacing at respective bundle branches 112 in the septum 104. For example, an active electrode of the pacing element 108 can be positioned at the left bundle branch 114 in the septum 104. Similarly, the fixation element 106 can be positioned at the right bundle branch 116 in the septum 104. Optionally, one of the elements may be at a bundle branch and the other element may not be at a bundle branch.

Referring to FIG. 2, a side view of a biostimulator is shown in accordance with an embodiment. The biostimulator 100 can be a leadless cardiac pacemaker that can perform cardiac pacing and that has many of the advantages of conventional cardiac pacemakers while extending performance, functionality, and operating characteristics. The biostimulator 100 can have two or more electrodes, e.g., a portion of the pacing element 108 that acts as an active electrode and/or a portion of the fixation element 106 or a housing 202 that acts as an active electrode. The electrodes can deliver pacing pulses to bundle branches 112 within the septum 104 of the heart 102 to perform deep septal pacing, and optionally, can sense electrical activity from the muscle. The electrodes may also communicate bidirectionally with at least one other device within or outside the body.

In an embodiment, the biostimulator 100 includes the housing 202 having a longitudinal axis 204. The housing 202 can contain a primary battery to provide power for pacing, sensing, and communication, which may include, for example, bidirectional communication. The housing 202 can optionally contain an electronics compartment 206 (shown by hidden lines) to hold circuitry adapted for different functionality. For example, the electronics compartment 206 can contain circuits for sensing cardiac activity from the electrodes, circuits for receiving information from at least one other device via the electrodes, circuits for generating pacing pulses for delivery to tissue via the electrodes, or other circuitry. The electronics compartment 206 may contain circuits for transmitting information to at least one other device via the electrodes and can optionally contain circuits for monitoring device health. The circuit of the biostimulator 100 can control these operations in a predetermined manner. The biostimulator 100 can perform leadless pacing without a pulse generator located in the pectoral region or abdomen and/or without an electrode-lead separate from the pulse generator. The biostimulator 100 may also lack a communication coil or antenna, and may not have the substantial battery power required for transmitted communication through a communication coil or antenna.

Leadless pacemakers or other leadless biostimulators 100 can be fixed to an intracardial implant site, e.g., at the septal wall 110, by one or more actively engaging mechanisms or fixation mechanisms, such as a screw or helical member that screws into the myocardium. In an embodiment, the biostimulator 100 includes the fixation element 106 coupled to the housing 202. The fixation element 106 can include a helical fixation element and/or several tines, as described below. More particularly, the biostimulator 100 can include a header assembly having a flange 208 coupled to a distal housing end 209 of the housing 202, and the fixation element 106 can be coupled to and extend distal to the flange 208. The fixation element 106 can extend about the longitudinal axis 204. For example, in the case of a helical fixation element, the element can spiral about the longitudinal axis to a distal tip. In the case of a fixation element 106 having several tines, the tines can be arranged about the longitudinal axis and extend to respective distal tips. Accordingly, when the biostimulator 100 is delivered to the target tissue, the distal tip(s) of the fixation element 106 can pierce the tissue and the housing 202 can be rotated or pushed to affix the fixation element 106 to the target tissue.

In an embodiment, the biostimulator 100 includes an attachment feature 210. The attachment feature 210 can be mounted on a proximal housing end 213 of the housing 202. More particularly, the attachment feature 210 can be mounted on an opposite end of the housing 202 from the fixation element 106 and the pacing element 108, which as described above, can be coupled to the distal housing end 209 of the housing 202. The attachment feature 210 can facilitate precise delivery or retrieval of the biostimulator 100. For example, the attachment feature 210 can be formed from a rigid material to allow a transport system to engage the attachment feature 210 and transmit torque and/or axial loads to the attachment feature 210 to plunge the fixation element 106 or the pacing element 108 into the target tissue.

The biostimulator 100 can include the pacing element 108 coupled to the housing 202. The pacing element 108 can be coaxially arranged with the fixation element 106 about the longitudinal axis 204. More particularly, the pacing element 108 can extend along, e.g., axially along or helically about, the longitudinal axis 204 at a location that is radially inward from the fixation element 106.

The pacing element 108 can, like the fixation element 106, affix or anchor in the target tissue. For example, the pacing element 108 may include a helical element extending distally to a distal tip having a distal pacing point. The distal pacing point of the pacing element 108 may be distal to a distal fixation tip of the fixation element 106. Both the fixation element 106 and the pacing element 108 can include a helical element to screw into a target tissue or a conical element to pierce into the target tissue. The fixation element 106 and/or the pacing element 108 can engage the tissue, and the housing 202 can be advanced and/or rotated to cause the distal pacing point of the pacing element to pierce the tissue and anchor the biostimulator 100. Accordingly, both the fixation element 106 and the pacing element 108 may be referred to as fixation elements, which are protected from the target tissue by a protective sleeve as described below.

Referring to FIG. 3, a perspective view of a biostimulator system is shown in accordance with an embodiment. As described above, the biostimulator 100 can be delivered to or retrieved from a patient using delivery or retrieval systems. The delivery or retrieval systems, which may be catheter-based systems used to carry a leadless biostimulator intravenously to or from a patient anatomy. The delivery or retrieval systems may be referred to collectively as transport systems, or biostimulator transport systems.

A biostimulator system 300 can include a biostimulator transport system 302. The biostimulator 100 can be attached, connected to, or otherwise mounted on the biostimulator transport system 302. For example, the biostimulator transport system 302 can include a catheter shaft 304 extending to a distal shaft end 306, and the biostimulator 100 can be mounted on the catheter shaft 304. More particularly, the biostimulator transport system 302 can include a biostimulator coupling 308 mounted on the distal shaft end 306 to receive the biostimulator 100 having a fixation element (e.g., the fixation element 106 and/or the pacing element 108). The biostimulator 100 can thereby be advanced intravenously into or out of the heart 102.

The biostimulator transport system 302 can include a handle 310 to control movement and operations of the transport system from outside of a patient anatomy. One or more elongated members can extend distally from the handle 310. For example, the catheter shaft 304 can extend distally from the handle 310. The catheter shaft 304 can extend to the biostimulator coupling 308 at a distal end of the transport system.

The biostimulator transport system 302 can include an outer sheath 312. The outer sheath 312 can include a protective sleeve 314. The protective sleeve 314, as described below, can cover the biostimulator 100 and/or the biostimulator coupling 308 during delivery and implantation. The outer sheath 312 can extend over, and be longitudinally movable relative to, the catheter shaft 304. The biostimulator transport system 302 may also include an introducer sheath 316 that can extend over, and be longitudinally movable relative to, the outer sheath 312. The introducer sheath 316 can cover a distal end of the outer sheath 312, the catheter shaft 304, the biostimulator coupling 308, and/or the biostimulator 100 as those components are passed through an access device into the patient anatomy.

Several components of the biostimulator transport system 302 are described above by way of example. It will be appreciated, however, that the biostimulator transport system 302 may be configured to include additional or alternate components. More particularly, the biostimulator transport system 302 may be configured to deliver and/or retrieve the biostimulator 100 to or from the target anatomy. Delivery and/or retrieval of the biostimulator 100 can include retaining the biostimulator during transport to the target anatomy and rotation of the biostimulator during implantation of the biostimulator at the target anatomy. For example, retention and/or rotation of the biostimulator 100 may be facilitated by the biostimulator coupling 308. Accordingly, the biostimulator transport system 302 can incorporate features to retain and rotate the biostimulator 100.

The protective sleeve 314 can be a distal portion of the outer sheath 312 used to cover a distal portion of the biostimulator system 300. For example, the protective sleeve 314 can cover all or a portion of the biostimulator 100 and/or the biostimulator 308. The protective sleeve 314 can cover and protect the fixation element(s) 106/108 during delivery to protect tissue from being punctured by a distal tip of the fixation element. The protective sleeve 314, when covering the fixation element, is in a protective state. When the catheter is positioned near the target tissue, e.g., in the right ventricle, the protective sleeve 314 can be pulled back to expose the biostimulator 100 and to allow the fixation element to pierce the target tissue to anchor the biostimulator for pacing. The protective sleeve 314, when not covering the fixation element, is in an unprotective state. Accordingly, the protective sleeve 314 is movable relative to the biostimulator 100 and/or biostimulator coupling 308 between the protective state and the unprotective state.

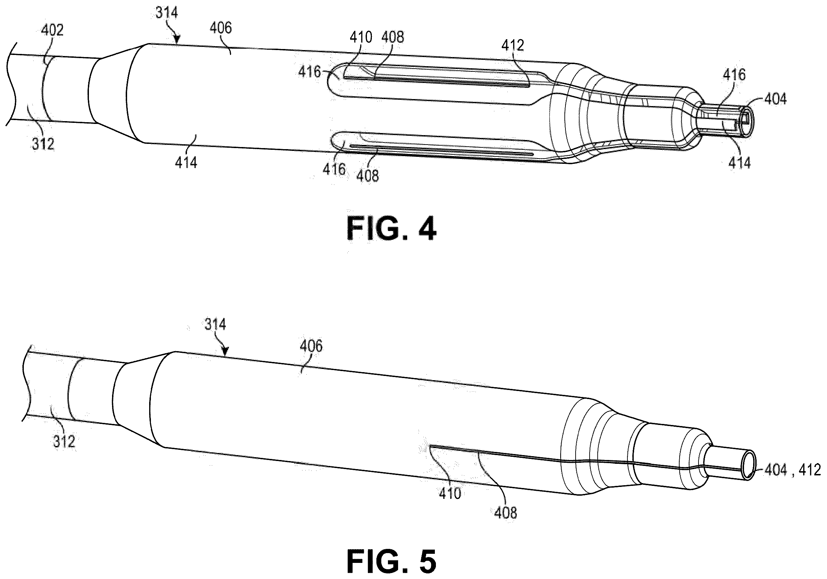

Referring to FIG. 4, a perspective view of a protective sleeve of a biostimulator transport system is shown in accordance with an embodiment. The protective sleeve 314 can be mounted on a tubular shaft of the outer sheath 312. More particularly, the protective sleeve 314 can have a proximal sleeve end 402 connected to the tubular shaft, and can extend from the proximal sleeve end 402 to a distal sleeve end 404.

In an embodiment, the protective sleeve 314 includes a sleeve body 406 shaped to conform to the biostimulator 100 when covering the biostimulator 100. For example, the sleeve body 406 can taper outward from the proximal sleeve end 402 to a larger diameter that conforms to the housing 202 of the biostimulator 100. Similarly, near the distal sleeve end 404, the sleeve body 406 can taper inward to conform to a tapered strain relief or fixation element at a distal end of the biostimulator 100. The distal taper may have one or more taper sections terminating at the distal sleeve end 404. The sleeve body 406 may therefore cover the biostimulator 100 without substantially increasing a crossing profile of the biostimulator system 300.

In an embodiment, the protective sleeve 314 includes one or more slits 408. The slits 408 can expand when the protective sleeve 314 moves from the protective state to the unprotective state. More particularly, the slits 408 can form expandable regions that flex and open when the protective sleeve 314 is retracted over the biostimulator 100. The slits 408 can therefore widen to permit communication between the biostimulator 100 and an external communication device via fluid that is in contact with the housing 202 through the slits 408.

Each slit 408 can be located along a medial section of the protective sleeve 314, e.g., proximal to the distal tapering section. The slit 408 can extend along the sleeve body 406 from a proximal slit end 410 to a distal slit end 412. The distal slit end 412 can be proximal to the distal sleeve end 404 of the sleeve body 406. For example, the slit 408 can terminate at the distal slit end 412 proximal to the tapering section that covers the fixation element 106 in the protective state.

The sleeve body 406 may have a composite structure, including a rigid section 414 and a flexible section 416 The rigid section 414 may be flexible, but may be more rigid than the flexible section 416. The rigid section 414 can therefore stabilize the sleeve structure, and support the flexible section 416. In an embodiment, the slit 408 is located in the flexible section 416. Accordingly, the slit material can be flexible and capable of being deformed from a closed state to an open state, as described below.

The flexible section 416 can extend in striations longitudinally to the distal sleeve end 404. The striations can be circumferentially separated by striations of the rigid section 414. The flexible striations can stretch, e.g., when the tapering section is withdrawn over the housing 202 of the biostimulator 100, to allow the distal portion of the sleeve body 406 to retract over the housing 202 from the protective state to the unprotective state.

Several embodiments, including the embodiment shown in FIG. 4, include slits that can open or close when the protective sleeve 314 transitions from the protective state to the unprotective state, and vice versa. In an embodiment, a membrane (not shown) is mounted on an outer surface of the protective sleeve 314 to cover the slits such that the openings formed by the slits during opening and closing are not exposed to a surrounding environment. For example, an external tubular membrane can be mounted on the outer surface of the protective sleeve 314 over the slit 408. The membrane can extend over the slit 408 and cover the slit from the proximal slit end 410 to the distal slit end 412. The membrane can surround the protective sleeve 314 circumferentially, and may be formed from an elastomeric polymer such that the membrane can expand to allow the slits to open when the sleeve is retracted. Accordingly, the slits can be covered during transition to and from the protective state, preventing ingress of structures such as valve leaflets or other tissue of the target anatomy.

Referring to FIG. 5, a perspective view of a protective sleeve of a biostimulator transport system is shown in accordance with an embodiment. The one or more slits 408 of the protective sleeve 314 may, rather than terminating proximal to the distal sleeve end 404, extend along the sleeve body 406 from the proximal slit end 410 to the distal slit end 412 at the distal sleeve end 404 of the sleeve body 406. More particularly, the slit 408 can terminate at the distal sleeve end 404 to form a slit end of the sleeve body 406.

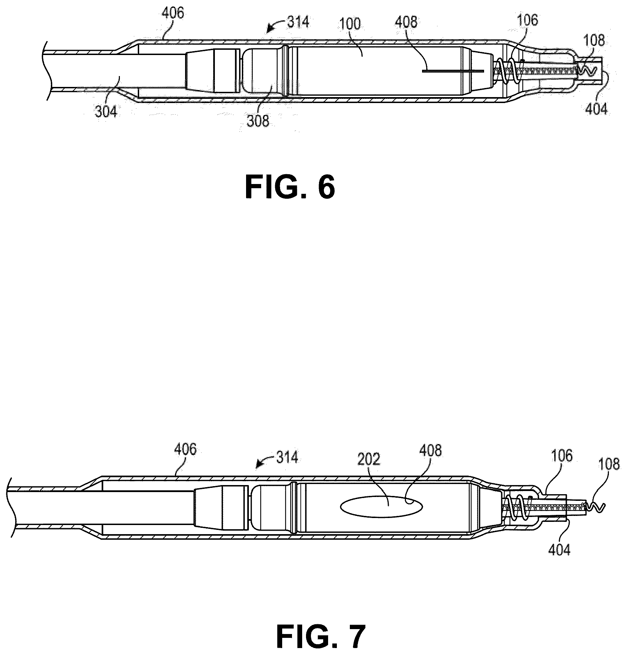

Referring to FIG. 6, a side view of a protective sleeve of a biostimulator transport system in a protective state is shown in accordance with an embodiment. In the protective state, the distal sleeve end 404 is distal to the fixation element (the pacing element 108 used to pierce and anchor in the target tissue). As described above, the sleeve body 406 conforms to the underlying catheter and biostimulator components, including the catheter shaft 304, the biostimulator coupling 308, and the biostimulator 100.

The slit 408 can be in a closed state when the sleeve body 406 is forward over the fixation element. For example, the preformed profile of the sleeve body 406, including optionally the rigid section 414 supporting the flexible section 416 having the slit 408, can match the profile of the underlying components and can therefore have no expansive stresses applied therefrom. In the closed state, the sleeve body 406 conforms to and surrounds the underlying components and protects the fixation element from snagging tissue during delivery to a target site.

Referring to FIG. 7, a side view of a protective sleeve of a biostimulator transport system in an unprotective state is shown in accordance with an embodiment. In the unprotective state, the distal sleeve end 404 is retracted proximal to the fixation element. The transition in state can occur when the catheter shaft 304 is pushed forward to allow the helical element of the fixation element 106 to emerge from an interior lumen of the sleeve body 406 and become exposed to the surrounding environment. When the tapered section of the sleeve body 406 is pulled over the housing 202 of the biostimulator 100, an expansive stress can be applied to the sleeve body 406. The stress can produce a deformation, e.g., radial expansion, of the slit portion of the sleeve body 406. Accordingly, the slit 408 can transition to the open state.

In the open state, the slit 408 provides a window between the housing 202 of the biostimulator 100 and the surrounding environment. More particularly, the open slit 408 establishes a direct fluid path from a proximal portion of the housing 202 to the surrounding environment. The proximal portion of the housing 202 can surround communicative elements of the biostimulator 100, such as an antenna or region used to conduct electrical communication signals. Accordingly, communication signals can be delivered directly from the housing 202 through the fluid pathway to the surrounding environment. More particularly, the open slit 408 permits communication to occur more effectively. The sleeve body 406 therefore protects the fixation element 106 in the protective state, but facilitates anchoring of the biostimulator 100 and communication with external devices in the unprotective state.

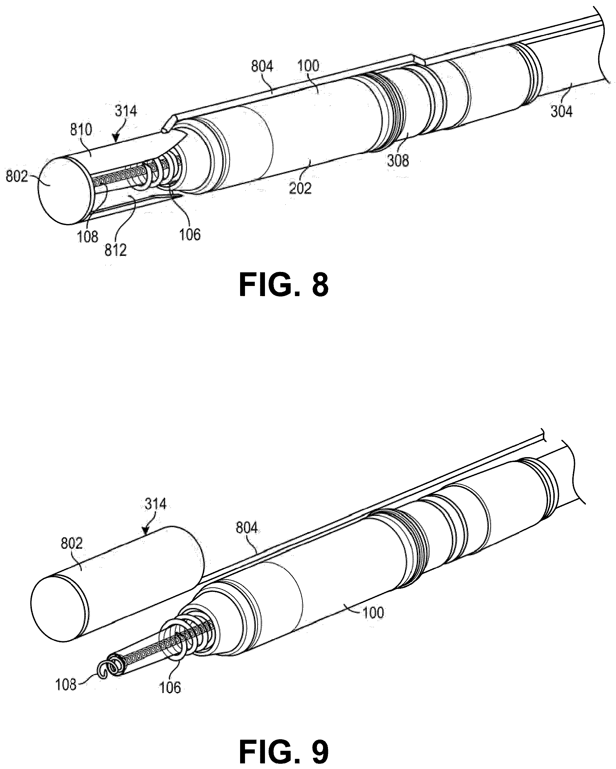

Referring to FIG. 8, a perspective view of a protective sleeve of a biostimulator transport system in a protective state is shown in accordance with an embodiment. The protective sleeve 314 can minimize a covering of non-piercing portions of the biostimulator 100, such as the housing 202. In an embodiment, the protective sleeve 314 includes a cap 802 mounted on a wire 804. The cap 802 can be sized and shaped to receive the fixation element(s) of the biostimulator 100, including the pacing element 108 and/or the fixation element 106. A portion of the biostimulator 100 proximal to the fixation element(s) may, however, remain uncovered in the protective state. For example, the housing 202, as well as the biostimulator coupling 308 and the catheter shaft 304 of the biostimulator transport system 302, may be exposed to a surrounding environment.

The cap 802 can include a sidewall 810 having a window 812. For example, the sidewall 810 can be a tubular section terminating at a flat end wall. The window 812 can be a port or opening extending laterally through the sidewall 810. The window 812 therefore allows the biostimulator 100 to be exposed laterally, even in the protective state. The cap 802, however, can cover the fixation element(s), or at least the piercing tip(s) of the fixation element(s), in the protective state. Accordingly, the fixation element(s) can be protected from piercing tissue during delivery, but exposed portions of the biostimulator 100 can communicate effectively through an adjacent fluid. Additionally, the minimal coverage of the biostimulator 100 can reduce a stiffness of the biostimulator system 300, allowing the system to more easily track or steer to an implantation site.

Referring to FIG. 9, a perspective view of a protective sleeve of a biostimulator transport system in an unprotective state is shown in accordance with an embodiment. The protective sleeve 314 can be moved to the unprotective state by rotating the wire 804. The wire 804 can be a mandrel or torque shaft for transmitting torque from a proximal wire end to a distal wire end connected to the cap 802. For example, the wire 804 can be twisted at the proximal wire end, and torque can be transmitted to cause the cap 802, which is connected to the distal wire end, to rotate about a wire axis. When the wire 804 is rotated, the fixation element 106 can pass through the window 812. The cap 802 may therefore rotate away from the biostimulator 100 from the protective state to the unprotective state. In the unprotective state, the fixation element 106 can be exposed and capable of being engaged and anchored in the target tissue.

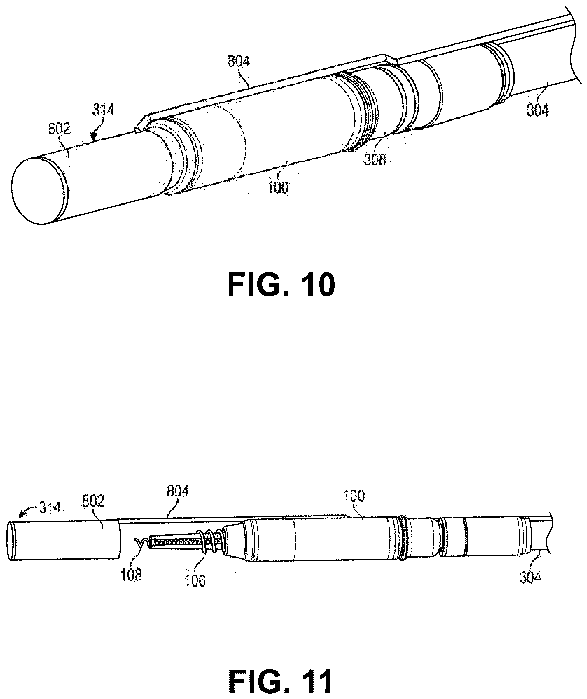

Referring to FIG. 10, a perspective view of a protective sleeve of a biostimulator transport system in a protective state is shown in accordance with an embodiment. The window cavity may be omitted, in an embodiment. For example, the protective sleeve 314 can include the cap 802 that is pushed forward and rotated by the wire 804 to transition from the protective state to the unprotective state. In the protective state, the cap 802 can cover and entirely surround the distal portion of the biostimulator 100 have the fixation element. The housing 202 of the biostimulator 100 may, however, be exposed. The fixation element can be sheathed, therefore, without impeding steering or communication of the biostimulator system 300.

Referring to FIG. 11, a side view of a protective sleeve of a biostimulator transport system in an unprotective state is shown in accordance with an embodiment. To transition to the unprotective state, the wire 804 can be pushed forward to move the cap 802 distally beyond the fixation element. When distally displaced, the cap 802 can no longer cover the fixation element.

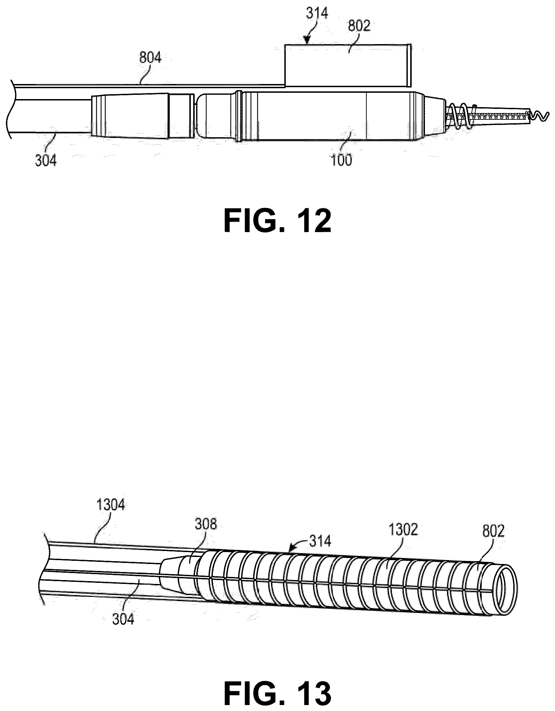

Referring to FIG. 12, a side view of a protective sleeve of a biostimulator transport system in an unprotective state is shown in accordance with an embodiment. The wire 804 may be rotated to move the cap 802 laterally away from a biostimulator axis. When the cap 802 is rotated away from the fixation element, the wire 804 can then be pulled to retract the cap 802 proximal to the fixation element. The fixation element may be advanced to pierce and anchor at the target tissue site.

Referring to FIG. 13, a perspective view of a protective sleeve of a biostimulator transport system in a protective state is shown in accordance with an embodiment. The protective sleeve 314 can be collapsible to transition between the protective state and the unprotective state. For example, when collapsed, the protective sleeve 314 can expose the underlying biostimulator 100 without necessarily retracting over the biostimulator coupling 308.

In an embodiment, the protective sleeve 314 has a corrugated wall 1302 to contract when the protective sleeve 314 moves from the protective state to the unprotective state. The corrugated wall 1302 can have a composite structure, including supportive elements and a flexible skin. For example, the protective sleeve 314 may have one or more rings, e.g., metallic rings, connected by a webbing of a polymer material. The rings made provide the supportive structure. Alternatively, a spring may be used to support the webbing. In either case, the supportive structure can provide radial stiffness to the structure while allowing longitudinal deformation of the protective sleeve 314. More particularly, the corrugated wall 1302 can shorten when a longitudinal compressive load is applied. Optionally, the supportive structure can provide a restorative force when the compressive load is removed. Accordingly, the protective sleeve 314 can be pulled or pushed to a shorter, unprotective state, and can then return to the extended, protective state.

The compressive load that transitions the protective sleeve 314 to the unprotective state may be applied by one or more pull wires 1304. For example, there may be two or more, e.g., three, pull wires 1304 circumferentially distributed about the catheter shaft 304. The circumferential distribution of the pull wires 1304 can be uniform to ensure that application of the pull force is evenly distributed around the corrugated wall 1302. Each pull wire 1304 can connect to the protective sleeve 314 at a location distal to the biostimulator coupling 308. Accordingly, when a pulling force is applied to the pull wire 1304, the corrugated wall 1302 can be compressed between the biostimulator coupling 308 and an attachment point of the pull wire 1304, and the protective sleeve 314 can shorten.

The pull wires 1304 may extend external to the catheter shaft 304, as shown in FIG. 13. Alternatively, the wires 1304 can extend through an inner diameter of the catheter shaft 304, or through lumens formed in the wall of the catheter shaft 304. In any case, the pull wires 1304 may extend proximally two the handle 310 to allow a user to apply the pull force to the wires 1304.

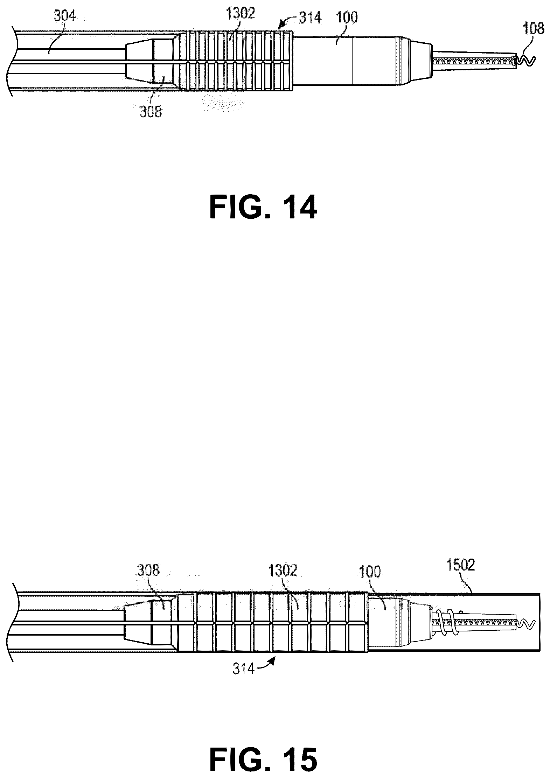

Referring to FIG. 14, a side view of a protective sleeve of a biostimulator transport system in an unprotective state is shown in accordance with an embodiment. In the unprotective state, the corrugated wall 1302 is shortened to expose the biostimulator 100. More particularly, the fixation element of the biostimulator 100 is exposed to allow the biostimulator 100 to engage and anchor in the target tissue. The biostimulator 100 may then be released and the protective sleeve 314 can return to the extended state, e.g., the original position, and removed from the patient anatomy.

Referring to FIG. 15, a side view of a protective sleeve of a biostimulator transport system in a protective state is shown in accordance with an embodiment. The protective sleeve 314 can include a tubular wall 1502 coupled to the corrugated wall 1302. The tubular wall 1502 may lack corrugations, and may therefore be more rigid than the corrugated wall 1302. More particularly, the rigid tubular wall 1502 can resist extension or contraction. In the protective state, the tubular wall 1502 can extend over a distal portion of the biostimulator 100. The corrugated wall 1302 can cover a proximal portion of the biostimulator 100 and/or the biostimulator coupling 308. Advantageously, the fixation element 106 of the biostimulator 100 may be distal to the corrugated wall 1302 and, thus, a risk of the helices getting caught on the compression rings/spring or webbing may be reduced.

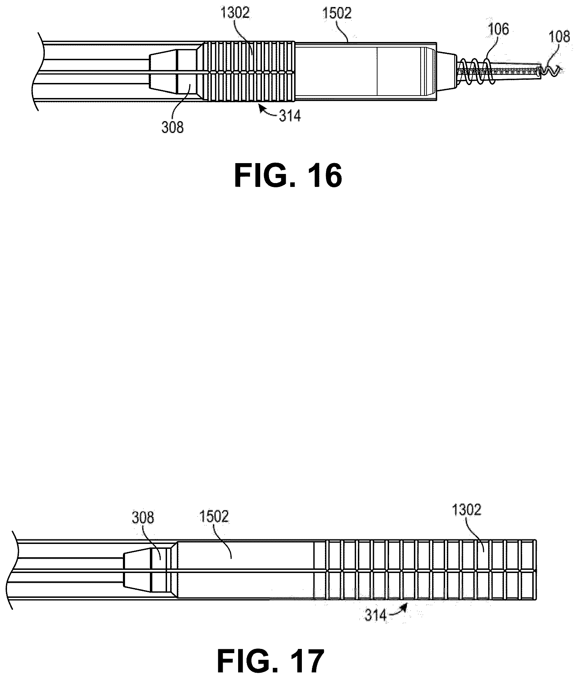

Referring to FIG. 16, a side view of a protective sleeve of a biostimulator transport system in an unprotective state is shown in accordance with an embodiment. In the unprotective state, the tubular wall 1502 can retract to a position that exposes the fixation element 106. The tubular wall 1502 may cover a distal portion of the biostimulator 100, and the compressed corrugated wall 1302 can cover a proximal portion of the biostimulator 100 and/or the biostimulator coupling 308. The biostimulator 100 can engage the target tissue and be released from the biostimulator transport system 302.

Referring to FIG. 17, a side view of a protective sleeve of a biostimulator transport system in a protective state is shown in accordance with an embodiment. The non-compressible tubular section may cover a proximal portion of the biostimulator 100 in the protective state. More particularly, the tubular section can cover and extend distally from the biostimulator coupling 308 to a distal tube end. The corrugated wall 1302 can connect to the distal tube end and extend distally to a distal end that is distal to the fixation element 106. Accordingly, the protective sleeve 314 can cover the biostimulator 100 entirely in the protective state.

The corrugated tube can have rings and or spring supportive elements, as described above. Alternatively, a fiber mesh may be incorporated in the webbing to provide structure to the corrugated wall 1302. The fiber mesh may be flexible to limit an impact on steering. More particularly, the accordion sleeve having the corrugated wall 1302 distal to the tubular wall 1502 may have a more flexible and steerable distal portion.

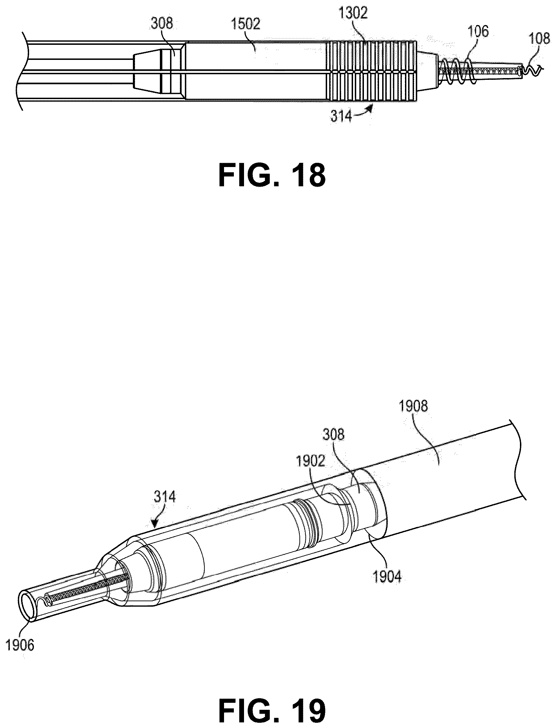

Referring to FIG. 18, a side view of a protective sleeve of a biostimulator transport system in an unprotective state is shown in accordance with an embodiment. In the unprotective state, the corrugated wall 1302 can retract to a position that exposes the fixation element 106. The compressed corrugated wall 1302 may cover a distal portion of the biostimulator 100, and the tubular wall 1502 can cover a proximal portion of the biostimulator 100 and/or the biostimulator coupling 308. The biostimulator 100 can engage the target tissue and be released from the biostimulator transport system 302.

Referring to FIG. 19, a perspective view of a protective sleeve of a biostimulator transport system in a protective state is shown in accordance with an embodiment. The protective sleeve 314 may be invertible to fold on itself when the protective sleeve moves from the protective state to the unprotective state. In an embodiment, the protective sleeve 314 can be formed from a flexible, stretchy material. For example, the sleeve may be formed from an elastomeric polymer. The protective sleeve 314 may extend from a first sleeve end 1902 to a second sleeve end 1904 through a foldback 1906. For example, the protective sleeve 314 can have a first section extending between the first sleeve end 1902 and the foldback 1906 and a second section extending between the second sleeve end 1904 and the foldback 1906. The flexible sleeve inverts on itself and the foldback 1906, which may be located distal to the fixation element in the protective state, can move when the second sleeve end 1904 moves relative to the first sleeve end 1902.

The biostimulator transport system 302 can include an outer catheter shaft 1908, which may extend around the catheter shaft 304. More particularly, the catheter shaft 304 and the outer catheter shaft 1908 may be coaxial and concentric, and the outer catheter shaft 1908 may radially surround the catheter shaft 304. In an embodiment, the first sleeve end 1902 can be coupled to the catheter shaft 304 and/or the biostimulator coupling 308, and the second sleeve end 1904 can be coupled to the outer catheter shaft 1908. Accordingly, the second sleeve end 1904 can be radially outward of the first sleeve end 1902. Movement of the outer catheter shaft 1908 in a longitudinal direction relative to the catheter shaft 304 can move the second sleeve end 1904 longitudinally relative to the first sleeve end 1902.

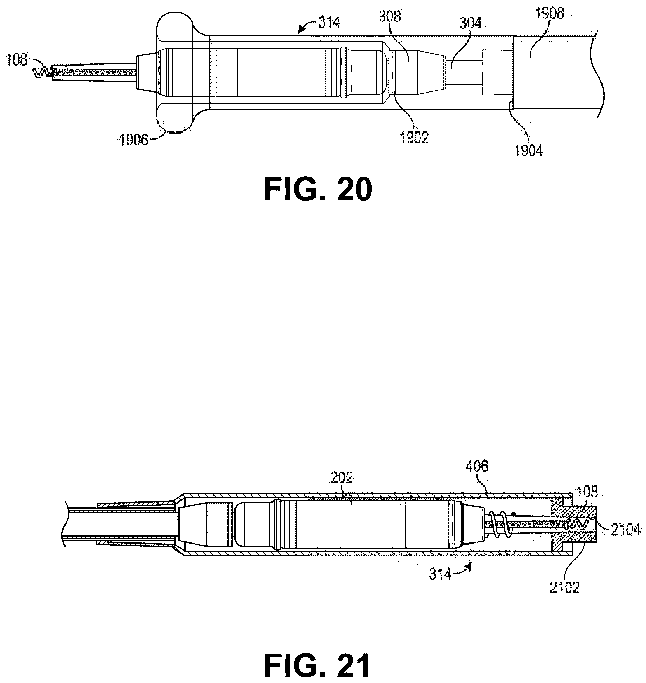

Referring to FIG. 20, a side view of a protective sleeve of a biostimulator transport system in an unprotective state is shown in accordance with an embodiment. When the outer catheter shaft 1908 is retracted relative to the catheter shaft 304, the protective sleeve 314 can roll backward to expose the fixation element of the biostimulator 100. The first, inner section of the folded protective sleeve 314 can have an inner surface that presses against the biostimulator 100 in the protective state. In the unprotective state, the surface can flip outward, facing away from the biostimulator 100. Accordingly, the foldback 1906, which is initially distal to the fixation element, can move proximal to the fixation element. The peeling sleeve can fit closely against the biostimulator 100 and, therefore, can provide for a low crossing profile that conforms to any shape of the biostimulator 100 and/or fixation element(s). The protective sleeve 314 can be pushed forward, e.g., by translating the outer catheter shaft 1908 forward, to return the sleeve to the protective state.

Referring to FIG. 21, a side view of a protective sleeve of a biostimulator transport system in a protective state is shown in accordance with an embodiment. The protective sleeve 314 can incorporate a semi-flexible protective sleeve 314 having a soft and compliant seal. In an embodiment, the protective sleeve 314 includes the sleeve body 406 containing a compliant plug 2102 through which the biostimulator 100 passes when the protective sleeve 314 moves from the protective state to the unprotective state. For example, the sleeve body 406 can have a cavity storing the biostimulator 100, and the compliant plug 2102 may be loaded in the cavity distal to the housing 202. The compliant plug 2102 may be molded into the protective sleeve 314 or attached in the cavity by a subsequent manufacturing operation.

In an embodiment, the compliant plug 2102 has a central channel 2104. The central channel 2104 can be sized to receive a portion of the biostimulator 100. For example, the fixation element 106 may be located in the central channel 2104 in the protective state. When the biostimulator transport system 302 is located in the target anatomy, e.g., the right ventricle, the biostimulator 100 may be pushed forward to expose the fixation element 106 to the surrounding environment.

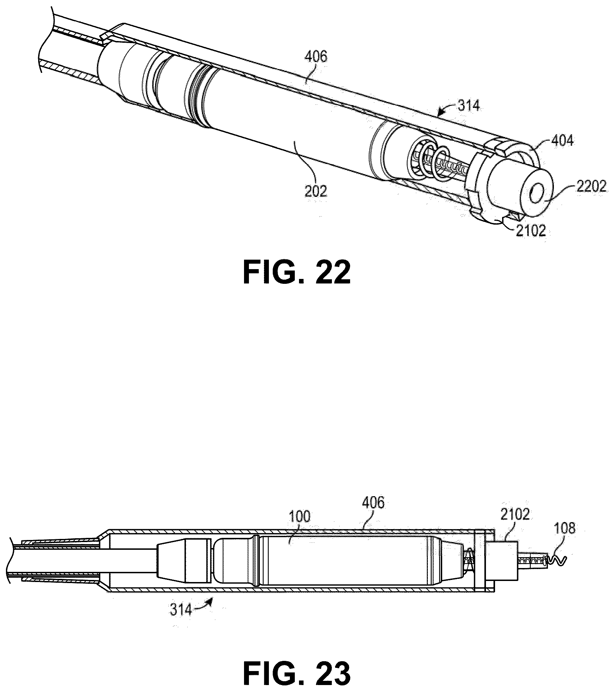

Referring to FIG. 22, a perspective view of a protective sleeve of a biostimulator transport system in a protective state is shown in accordance with an embodiment. The compliant plug 2102 can have a distal plug end 2202 distal to a distal sleeve end 404 of the protective sleeve 314. For example, the distal plug end 2202 can protrude distally from the distal sleeve end 404. Accordingly, the compliant plug 2102, which may be formed from a softer material than the sleeve body 406, can act like a soft bumper that presses against the target tissue without causing trauma. The compliant plug 2102 may also provide visual feedback confirming that the distal plug end 2202 is pressed against the target tissue. For example, a length of the compliant plug 2102 may shorten, or a width of the compliant plug 2102 may widen, as the plug is compressed. Such deformation may be evident in a fluoroscopic image. Furthermore, one or more radiopaque markers may be mounted on compliant plug 2102, e.g., at a distal end and a proximal end of the plug, and the distance between the markers may be viewed to determine that the compliant plug 2102 is being compressed by the target tissue.

Referring to FIG. 23, a side view of a protective sleeve of a biostimulator transport system in an unprotective state is shown in accordance with an embodiment. When the compliant plug 2102 is pressed against the target tissue, the biostimulator 100 may be pushed forward through the compliant plug 2102. The biostimulator 100 passes through the central channel 2104 when the protective sleeve 314 moves from the protective state to the unprotective state. The biostimulator 100 can be pushed forward through the central channel 2104 in the compliant plug 2102, causing the plug to expand as the biostimulator 100 moves distally. The compliant plug 2102 can support and center a distal end of the biostimulator 100, e.g., the fixation element.

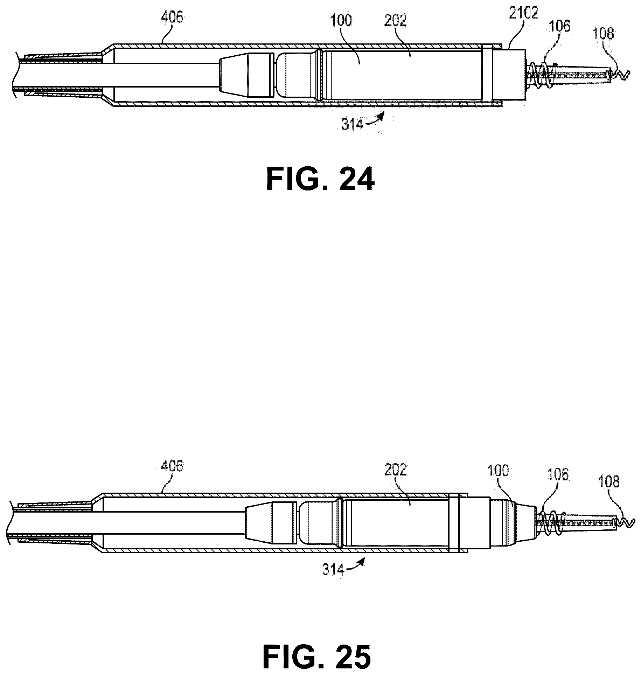

Referring to FIG. 24, a side view of a protective sleeve of a biostimulator transport system in an unprotective state is shown in accordance with an embodiment. As the biostimulator 100 is pushed further forward, both fixation elements, e.g., the fixation element 106 and the pacing element 108 can be exposed to the surrounding environment. The central channel 2104 within the compliant plug 2102 can expand more, as the housing 202 of the biostimulator 100 moves through the plug.

Referring to FIG. 25, a side view of a protective sleeve of a biostimulator transport system in an unprotective state is shown in accordance with an embodiment. When the protective sleeve 314 is in the unprotective state, the fixation element and a portion of the housing 202 may be exposed. The fixation element can be engaged with the target tissue, and implanted into the target tissue. The biostimulator 100 can be released from the biostimulator transport system 302, and the biostimulator transport system can be retracted from the patient anatomy.

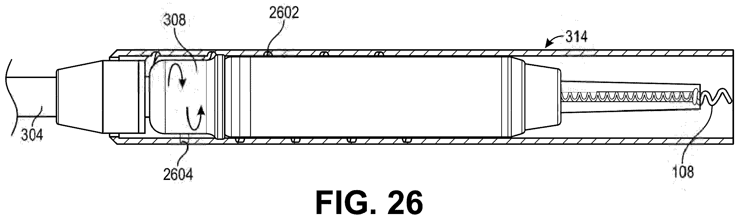

Referring to FIG. 26, a side view of a protective sleeve of a biostimulator transport system in a protective state is shown in accordance with an embodiment. The protective sleeve 314 may interact directly with other components of the biostimulator transport system 302 to actuate. For example, the protective sleeve 314 may include a thread 2602 engaging another component, such as the biostimulator coupling 308. Rotation of the other component, such as the biostimulator coupling 308, can move the protective sleeve 314 from the protective state to the unprotective state.

In an embodiment, the thread 2602 is an internal thread on an interior surface of the protective sleeve 314. The thread 2602 can engage a corresponding feature of the biostimulator coupling 308. For example, the biostimulator coupling 308 can include one or more knobs 2604 to engage the thread 2602. The knobs 2604 may be portions of a threaded structure, or may act as followers in a camming action along the thread 2602. Accordingly, when a torque shaft rotates the biostimulator coupling 308, the knobs 2604 can slide over the thread 2602 to drive the protective sleeve 314 in a longitudinal direction. Translation of the sleeve can expose the biostimulator fixation element in the unprotective state, or cover the biostimulator in the protective state.

Rotational movement of the biostimulator coupling 308 (or another component) can drive longitudinal movement of the protective sleeve 314. To achieve such action, however, rotational movement of the protective sleeve 314 may have to be resisted. In an embodiment, a protrusion may extend from a stationary component of the biostimulator transport system 302, such as the catheter shaft 304 or a bearing housing located between the catheter shaft 304 and the biostimulator coupling 308. The protrusion can engage a longitudinal groove in the interior surface of the protective sleeve 314. The protrusion can slide along the longitudinal groove, and can resist rotation of the protective sleeve 314. Accordingly, as the biostimulator coupling 308 rotates, the protective sleeve 314 can be driven longitudinally and remain fixed rotationally.

Actuation of the protective sleeve 314 via rotation of the biostimulator coupling 308, the torque shaft, the catheter shaft 304, etc., can remove the action of the physician that is typically required to displace the protective sleeve 314. More particularly, rather than applying separate longitudinal pushing or pulling inputs at the handle 310, the biostimulator 100 may be rotated to engage and drive the helical fixation element 106 into the target tissue in a usual manner. The rotation can, however, produce a corresponding retraction of the protective sleeve 314 such that the biostimulator 100 is exposed and driven into the target tissue using a same, rotational input at the handle 310. Accordingly, greater control of the movement and translation of the biostimulator 100, and a seamless deployment of the biostimulator 100 with minimal manual operations, may be achieved.

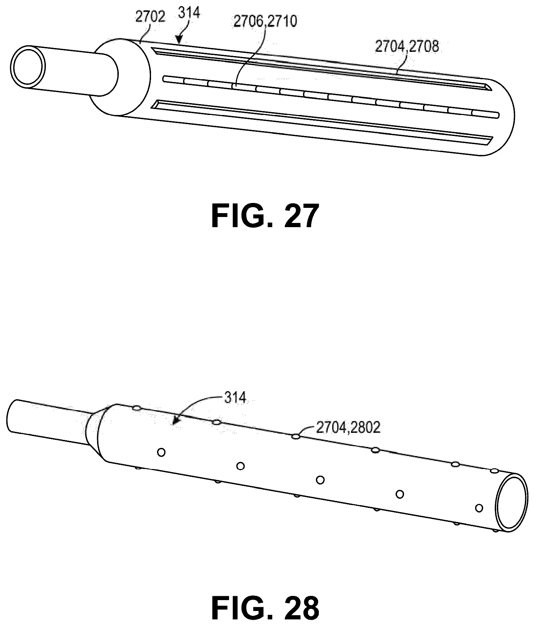

Referring to FIG. 27, a perspective view of a protective sleeve of a biostimulator transport system is shown in accordance with an embodiment. The protective sleeve 314 may be formed to reduce surface friction. Surface friction may exist between an outer surface of the protective sleeve 314 and a surrounding structure, such as a guide catheter or an anatomical structure. Accordingly, a contact area between the outer surface and the surrounding structure may be minimized to reduce the surface friction. In an embodiment, the protective sleeve 314 includes an outer sleeve surface 2702. The outer sleeve surface 2702 of the protective sleeve 314 can have one or more bosses 2704 or recesses 2706 that reduce the contact area.

Still referring to FIG. 27, the one or more bosses 2704 can include several ribs 2708. The ribs 2708 may extend longitudinally along the protective sleeve 314 and can be higher than (at a radially outward location relative to) an adjacent surface. For example, the ribs 2708 may be thin ridges. The ribs 2708 can project outward to provide rails for the surrounding structure and reduce surface contact with the protective sleeve 314.

The one or more recesses 2706 can include several grooves 2710. Like the ribs 2708, the grooves 2710 can reduce surface contact with surrounding structures. For example, the grooves 2710 may extend longitudinally along the protective sleeve 314 and can be lower than an adjacent surface. Accordingly, the adjacent surface may contact the surrounding structure while the grooved surfaces can remain out of contact, reducing contact surface area.

Referring to FIG. 28, a perspective view of a protective sleeve of a biostimulator transport system is shown in accordance with an embodiment. The one or more bosses 2704 can include several bumps 2802. The bumps 2802, like the ribs 2708, can be higher than an adjacent surface. Accordingly, the bumps 2802 can contact the surrounding structure while the adjacent surface may remain offset from the surrounding structure, reducing overall surface friction of the protective sleeve 314.

The surface features described above, which may be molded into the protective sleeve 314, can reduce surface friction and, optionally, may reduce a risk of tissue trauma. For example, the bosses 2704 and/or recesses 2706 may be distinct zones that are formed from softer material that is less traumatic to tissue. In an embodiment, a tip of the protective sleeve 314 is formed from a soft, e.g., elastomeric, polymer that reduces a possibility of trauma on the heart tissue and allows a physician to use the tip as a bumper or locating feature. Other features, such as radiopaque markers, may similarly be formed into the protective sleeve 314 to provide visual feedback when the protective sleeve 314 contacts the target tissue.

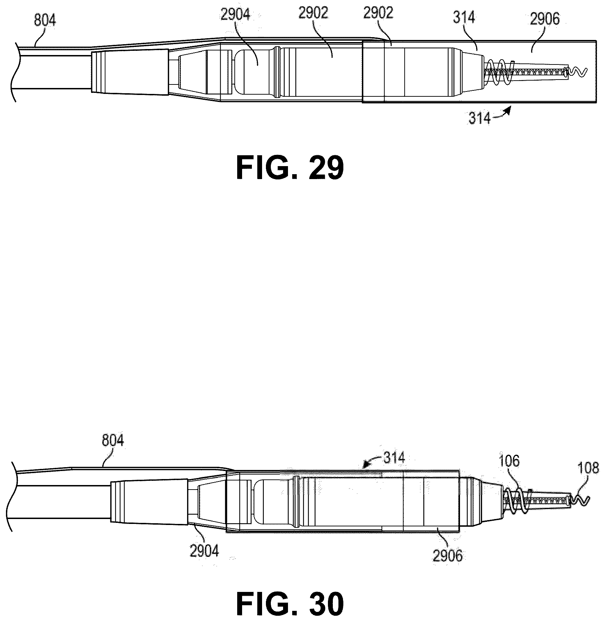

Referring to FIG. 29, a side view of a protective sleeve of a biostimulator transport system in a protective state is shown in accordance with an embodiment. The protective sleeve 314 can include one or more telescoping sections 2902 to contract or shorten when the protective sleeve 314 moves from the protective state to the unprotective state. For example, the telescoping section 2902 can include an outer segment that slides over an inner segment. In an embodiment, the outer segment can be a distal telescoping section 2906, and the inner segment can be a proximal telescoping section 2904. The proximal telescoping section 2904 can be mounted on the biostimulator coupling 308 and the distal telescoping section 2906 can be slidable on the proximal telescoping section 2904.

In the protective state, the distal telescoping section 2906 can be pushed forward, e.g., by the wire 804, to fully cover the fixation element 106 of the biostimulator 100. The proximal telescoping section 2904 can similarly cover a proximal portion of the biostimulator 100.

Referring to FIG. 30, a side view of a protective sleeve of a biostimulator transport system in an unprotective state is shown in accordance with an embodiment. To deploy the biostimulator 100, the wire 804 can be pulled to retract the distal telescoping section 2906 and expose the fixation elements 106 of the biostimulator 100. The proximal telescoping section 2904 can remain stationary and provide a guide over which the distal telescoping section 2906 tracks. When the biostimulator 100 is exposed, it may engage the target tissue to perform implantation. The telescoping sections 2902 can reduce a total length of the protective sleeve 314 and does not require the protective sleeve to be pulled back over the deflectable catheter shaft 304. Accordingly, the biostimulator transport system 302 can be flexible and steerable.

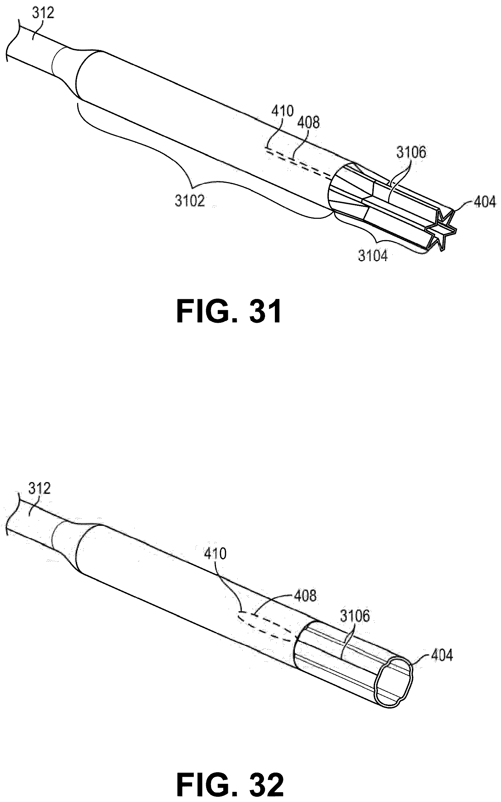

Referring to FIG. 31, a perspective view of a protective sleeve of a biostimulator transport system is shown in accordance with an embodiment. An alternative protective sleeve can include a folded or corrugated section that expands radially to the unprotective state and contracts radially to the protective state. The biostimulator transport system having the protective sleeve 314 including one or more folds, as described below, may have a similar catheter and/or biostimulator configuration as any of the embodiments described above. For example, the biostimulator transport system can include the catheter shaft extending to the distal shaft end, and the biostimulator coupling mounted on the distal shaft end to receive the biostimulator having the fixation element 106. FIGS. 31-32 omit such components to focus on the protective sleeve 314 component, however, it will be appreciated that the components may have similar configurations to those described and illustrated above.

In an embodiment, the protective sleeve 314 includes a proximal section 3102 and a distal section 3104. The proximal section 3102 may have a shape that conforms to the housing 202 of the biostimulator. The proximal section 3102 may be unfolded. For example, the proximal section 3102 may have a cylindrical tubular shape. Accordingly, in the protective state, the proximal section 3102 can contain the housing 202 of the biostimulator. The distal section 3104 can have a shape that conforms to the fixation element 106 of the biostimulator in the protective state, and by contrast to the proximal section 3102 , may include one or more folds to form a folded or corrugated section. Accordingly, the distal section 3104 of the protective sleeve 314 can cover the fixation element 106 in the protective state.

The one or more folds of the distal section 3104 can extend longitudinally. In an embodiment, several folds extend longitudinally, and the folds allow the distal section 3104 to collapse radially relative to the longitudinal axis in the protective state. The collapsed section can have a star-shaped profile. The star-shaped profile may extend from a transition point at a proximal end, at which the corrugated section transitions to the proximal section 3102, to the distal sleeve end. The length of the folded section may be sufficient to cover the fixation element 106 in the protective state.

In an embodiment, the protective sleeve 314 includes one or more slits 408. The slits 408 can extend proximally from the transition point along the proximal section 3102. For example, a slit 408 can extend longitudinally from the proximal end of the folded section to the proximal slit 408 end. The slit 408 may have a similar structure and function to that described above with respect to, e.g., FIGS. 6-7. More particularly, the one or more slits 408 can expand when the protective sleeve 314 moves from the protective state to the unprotective state (as shown in FIG. 32). Accordingly, the slit(s) 408 can improve wireless communication of the biostimulator in situ.

Referring to FIG. 32, a perspective view of a protective sleeve of a biostimulator transport system is shown in accordance with an embodiment. The protective sleeve 314 can be movable relative to the biostimulator coupling between the protective state and the unprotective state. Whereas the one or more folds of the corrugated section can allow for collapse of the distal section 3104 to conform to the fixation element 106 of the biostimulator in the protective state, in the unprotective state the corrugated section can expand radially to allow the biostimulator to pass distally toward the target anatomy. More particularly, when the biostimulator advances, the housing 202 of the biostimulator can force the one or more folds to open. The opening of the folds, e.g., an increase in an angle between adjacent surfaces of the folds, can cause the expanding corrugated section to take a form having a larger cross-sectional area. For example, the corrugated section can open to a cylindrical form similar to the cross-sectional shape of the proximal section 3102 of the protective sleeve 314. Accordingly, a cylindrical lumen can form to allow the biostimulator to pass distally.

In an embodiment, the distal section 3104 may have a softer material than the proximal section 3102. For example, the folded section may be formed from a lower durometer polymer than the proximal section 3102, and the sections may be fused at the transition point to form the protective sleeve 314. The transition in hardness can provide for a robust section covering the biostimulator during delivery, and a softer, atraumatic distal section 3104 leading the device through the target anatomy.

The protective sleeve 314 may be manufactured using several techniques. In an embodiment, the folded section is formed in a cylindrical tubing component. More particularly, the tubing component can be formed having a cylindrical profile, and the distal section 3104 of the tubing can be collapsed, e.g., squeezed, folded, etc., to form the corrugated section. The unfolded section may remain cylindrical, and provide the proximal section 3102 of the protective sleeve 314. In an alternative embodiment, the proximal section 3102 is formed from a folded tubing component. More particularly, the tubing component can be formed having a star-shaped profile, and the proximal section 3102 of the tubing can be expanded, e.g., blow-molded, heat set on a mandrel, etc., to form the cylindrical section. The folded section may remain star-shaped (or any other folded profile), and provide the distal section 3104 of the protective sleeve 314.

In the unprotective state, the protective sleeve 314 may not cover the fixation element 106. The one or more folds open when the protective sleeve 314 moves from the protective state to the unprotective state. Also, the one or more slits 408 can open as the folded section expands to have a larger cross-sectional area. Accordingly, the protective sleeve 314 can move relative to the biostimulator coupling between the protective state and the unprotective state to allow the biostimulator to engage the target anatomy and communicate wirelessly with a remote device.

Embodiments of biostimulator transport systems and methods of using such systems are described above. More particularly, such embodiments are described, either explicitly or implicitly. The following paragraphs summarize some of the described embodiments. More particularly, embodiments are described in the following paragraphs.

An embodiments relates toa biostimulator transport system comprising a catheter shaft extending to a distal shaft end, biostimulator coupling mounted on the distal shaft end to receive a biostimulator having a fixation element, and a protective sleeve movable relative to the biostimulator coupling between a protective state and an unprotective state The protective sleeve covers the fixation element in the protective state, and the protective sleeve does not cover the fixation element in the unprotective state. The protective sleeve includes one or more slits to expand when the protective sleeve moves from the protective state to the unprotective state.

In an embodiment, the one or more slits extend along a sleeve body from a proximal slit end to a distal slit end. In this embodiment, the distal slit end is proximal to a distal sleeve end of the sleeve body.

In an embodiment, the one or more slits extend along a sleeve body from a proximal slit end to a distal slit end at a distal sleeve end of the sleeve body.

An embodiment relates to a biostimulator transport system comprising a catheter shaft extending to a distal shaft end, a biostimulator coupling mounted on the distal shaft end to receive a biostimulator having a fixation element, and a protective sleeve movable relative to the biostimulator coupling between a protective state and an unprotective state. The protective sleeve covers the fixation element in the protective state, and the protective sleeve does not cover the fixation element in the unprotective state. The protective sleeve includes a cap mounted on a wire.

In an embodiment, the cap includes a sidewall having a window. In this embodiment, the fixation element passes through the window when the wire is rotated to move the cap from the protective state to the unprotective state.

An embodiment relates to a biostimulator transport system comprising a catheter shaft extending to a distal shaft end, a biostimulator coupling mounted on the distal shaft end to receive a biostimulator having a fixation element, and a protective sleeve movable relative to the biostimulator coupling between a protective state and an unprotective state. The protective sleeve covers the fixation element in the protective state, and the protective sleeve does not cover the fixation element in the unprotective state. The protective sleeve has a corrugated wall to contract when the protective sleeve moves from the protective state to the unprotective state.

In an embodiment, the protective sleeve includes a tubular wall coupled to the corrugated wall.

An embodiment relates to a biostimulator transport system comprising a catheter shaft extending to a distal shaft end, a biostimulator coupling mounted on the distal shaft end to receive a biostimulator having a fixation element, and a protective sleeve movable relative to the biostimulator coupling between a protective state and an unprotective state. The protective sleeve covers the fixation element in the protective state, and the protective sleeve does not cover the fixation element in the unprotective state. The protective sleeve is invertible to fold on itself when the protective sleeve moves from the protective state to the unprotective state.

In an embodiment, the biostimulator transport system further comprises an outer catheter shaft around the catheter shaft. In this embodiment, the protective sleeve has a first sleeve end coupled to the biostimulator coupling and a second sleeve end coupled to the outer catheter shaft.

An embodiment relates to a biostimulator transport system comprising a catheter shaft extending to a distal shaft end, a biostimulator coupling mounted on the distal shaft end to receive a biostimulator having a fixation element, and a protective sleeve movable relative to the biostimulator coupling between a protective state and an unprotective state. The protective sleeve covers the fixation element in the protective state, and the protective sleeve does not cover the fixation element in the unprotective state. The protective sleeve includes a sleeve body containing a compliant plug through which the biostimulator passes when the protective sleeve moves from the protective state to the unprotective state.

In an embodiment, the compliant plug has a central channel through which the biostimulator passes when the protective sleeve moves from the protective state to the unprotective state.

In an embodiment, the compliant plug has a distal plug end distal to a distal sleeve end of the sleeve body.

An embodiment relates to a biostimulator transport system comprising a catheter shaft extending to a distal shaft end, a biostimulator coupling mounted on the distal shaft end to receive a biostimulator having a fixation element, and a protective sleeve movable relative to the biostimulator coupling between a protective state and an unprotective state. The protective sleeve covers the fixation element in the protective state, and the protective sleeve does not cover the fixation element in the unprotective state. The protective sleeve includes a thread engaging the biostimulator coupling such that rotation of the biostimulator coupling moves the protective sleeve from the protective state to the unprotective state.

In an embodiment, the biostimulator coupling includes one or more knobs to engage the thread.

An embodiment relates to a biostimulator transport system comprising a catheter shaft extending to a distal shaft end, a biostimulator coupling mounted on the distal shaft end to receive a biostimulator having a fixation element, and a protective sleeve movable relative to the biostimulator coupling between a protective state and an unprotective state. The protective sleeve covers the fixation element in the protective state, and the protective sleeve does not cover the fixation element in the unprotective state. An outer sleeve surface of the protective sleeve has one or more bosses or recesses.

In an embodiment, the one or more bosses include a plurality of bumps.

In an embodiment, the one or more bosses include a plurality of ribs.

In an embodiment, the one or more recesses include a plurality of grooves.

An embodiment relates to a biostimulator transport system comprising a catheter shaft extending to a distal shaft end, a biostimulator coupling mounted on the distal shaft end to receive a biostimulator having a fixation element, and a protective sleeve movable relative to the biostimulator coupling between a protective state and an unprotective state. The protective sleeve covers the fixation element in the protective state, and the protective sleeve does not cover the fixation element in the unprotective state. The protective sleeve includes one or more telescoping sections to contract when the protective sleeve moves from the protective state to the unprotective state.

In an embodiment, the one or more telescoping sections includes a proximal telescoping section mounted on the biostimulator coupling and a distal telescoping section slidable on the proximal telescoping section.

An embodiment relates to a biostimulator transport system comprising a catheter shaft extending to a distal shaft end, a biostimulator coupling mounted on the distal shaft end to receive a biostimulator having a fixation element, and a protective sleeve movable relative to the biostimulator coupling between a protective state and an unprotective state. The protective sleeve covers the fixation element in the protective state, and the protective sleeve does not cover the fixation element in the unprotective state. The protective sleeve has a distal section including one or more folds that open when the protective sleeve moves from the protective state to the unprotective state.

In an embodiment, the biostimulator transport system further comprises one or more slits to expand when the protective sleeve moves from the protective state to the unprotective state.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator transport system (302), comprising:
a catheter shaft (304) extending to a distal shaft end (306);
a biostimulator coupling (308) mounted on the distal shaft end (306) to receive a biostimulator (100) having a fixation element (106); and
a protective sleeve (314) movable relative to the biostimulator coupling (308) between a protective state and an unprotective state, wherein the protective sleeve (304) covers the fixation element (106) in the protective state, wherein the protective sleeve (314) does not cover the fixation element (106) in the unprotective state, and wherein the protective sleeve (314) has a distal section (3104) including one or more folds that open when the protective sleeve (314) moves from the protective state to the unprotective state.

2. The biostimulator transport system of claim 1, wherein the one or more folds expand radially to the unprotective state, and wherein the folds contract radially to the protective state.

3. The biostimulator transport system of claims 1 or 2, wherein the protective sleeve (314) includes a proximal section (3102), and wherein the proximal section (3102) is unfolded.

4. The biostimulator transport system of any of claims 1-3, wherein, in the protective state, the distal section (3104) has a star-shaped profile.

5. The biostimulator transport system of claims 3 and 4, wherein the star-shaped profile extends from a transition point at the proximal section (3102) to a distal sleeve end (404) of the protective sleeve (314).

6. The biostimulator transport system of claims 3 or 5, wherein the distal section (3104) is formed from a softer material than the proximal section (3102).

7. The biostimulator transport system of claims 3, 5 or 6, wherein the protective sleeve (314) has a fusion between the distal section (3102) and the proximal section (3104).

8. The biostimulator transport system of any of claims 1-7, wherein the one or more folds extend longitudinally.

9. The biostimulator transport system of any of claims 1-8, further comprising one or more slits (408) to expand when the protective sleeve (314) moves from the protective state to the unprotective state.

10. The biostimulator transport system of claim 9, wherein the one or more slits (408) extend proximally from the one or more folds to a proximal slit end.

11. The biostimulator transport system of any of claims 1-10, wherein the distal section (3104) has a cross-sectional area, and wherein the cross-sectional area increases when the protective sleeve (314) moves to the unprotective state.

12. The biostimulator transport system of claim 11, wherein the one or more folds have adjacent surfaces, and wherein an angle between the adjacent surfaces increases when the protective sleeve (314) moves from the protective state to the unprotective state.

13. The biostimulator transport system of any of claims 1-12, wherein the distal section (3104) opens to a cylindrical form when the protective sleeve (3104) moves from the protective state to the unprotective state.

14. The biostimulator system of any of claims 1-13, further comprising the biostimulator (100) coupled to the biostimulator coupling (308), wherein the biostimulator (100) has the fixation element (106).

15. The biostimulator system of claim 14, wherein the fixation element (106) includes a helical fixation element (106).
